# EUROPEAN PATENT APPLICATION

(11) **EP 4 785 801 A1**
(43) Date of publication of application: **05.08.2026**
(21) Application number: 26156118.7
(22) Date of filing: 03.02.2026
(51) Int. Cl.: A23C 11/10, A23C 20/02, A23L 2/38, A23L 5/30, A23L 7/10, A23L 7/104, A23L 33/21, A23L 33/22, C12N 9/24

(54) **PRODUCING LIQUID OAT BASE AND PRODUCT FOR HUMAN CONSUMPTION**

(30) Priority: 04.02.2025 EP 25155827
(71) Applicant: Tetra Laval Holdings & Finance S.A., 1009 Pully (CH)
(72) Inventor: Funkquist, Ola, 221 86 Lund (SE); Cheaib, Rana, 221 86 Lund (SE); Lanzingh, Christer, 221 86 Lund (SE); Seah, Chong Meng, 629414 Singapore (SG)
(74) Representative: Tetra Pak Patent Attorneys

(57) **Abstract**

A method for producing a liquid oat base (7) for human consumption is disclosed. The method comprises processing (12, 34) oat raw material, water (2), and one or more enzymes (5) into a slurry, followed by hydrolyzing (18) the slurry for a defined period using the one or more enzymes (5). The hydrolyzed slurry undergoes high-pressure homogenization in a first homogenization step (20), followed by a second homogenization step (22) to achieve a uniform consistency. The one or more enzymes are deactivated (24), and the liquid oat base (7) is obtained (33) from the two-step homogenized slurry. The disclosure also includes a system for producing the liquid oat base and a method for producing an oat product from the base. The two-step homogenization enhances the textural properties, stability, and overall quality of the oat base while optimizing energy efficiency and resource utilization in the production process.

## Description

### Technical Field

The present disclosure generally relates to the field of oat base production for human consumption, and more particularly to a resource-efficient method and system for producing a liquid oat base.

### Background

Oats are a type of cereal grain and the edible seed of oat grass. Their consumption has increased significantly in recent years due to their health benefits, including cholesterol reduction, blood glucose control, and improved gut health. Compared to other grains, oats are also well-tolerated, even by individuals with celiac disease.

Oat-based beverages, commonly known as oat milk or oat drinks, are typically produced by soaking milled oat grains in water to extract nutrients. Enzymes may be added to hydrolyze gelatinized oat starch into dextrins, which are further broken down into simpler sugars. The liquid fraction is then separated from insoluble fiber, typically using a decanter or other centrifugation-based separation techniques. This liquid fraction, referred to as "liquid oat base," serves as a starting material for formulating oat-based beverages and other oat-derived products.

The conventional extraction process generates a substantial amount of solid residue, commonly referred to as "oat okara." While this by-product is sometimes repurposed as animal feed, it is often discarded, leading to significant environmental waste and disposal costs for the industry.

The residual fraction contains significant amounts of protein, fiber, fat, and other nutrients that could be valuable for human consumption. WO2023/021240 describes a technique for processing oat okara into an oat-based dispersion suitable for food product formulation. Although various embodiments for producing oat-based beverages are discussed, the focus is primarily on the beverage itself.

An equally important aspect in the production of oat-based products is the total production cost, which is becoming increasingly relevant due to rising energy and resource costs. Since the production of liquid oat base requires significant input of both energy and raw materials, optimizing the balance between resource utilization and product yield is crucial. Even a small improvement in process efficiency can result in substantial cost savings and sustainability benefits. Therefore, the specific method steps and equipment configuration used in the production of liquid oat base and other oat-based products are of growing importance, as they directly impact efficiency, scalability, and overall economic viability.

### Summary

One objective of the present invention to improve the prior art in respect of oat base manufacturing.

One such objective is to reduce the environmental impact and production costs associated with liquid oat-based food products, particularly by retaining most or all of the fibers from the oat raw material in the final product.

Another objective is to minimize the production of solid residue (oat okara) in the manufacturing of liquid oat base from oat kernels or whole oat flour, thereby reducing waste and improving resource efficiency.

A further objective is to optimize the balance between resource utilization and product yield in the production of a liquid oat base.

These objectives are achieved, in part, through a two-step homogenization process, which enhances the textural properties of the oat base while improving overall process efficiency.

One or more of these objectives, along with additional benefits that will become apparent from the description below, are at least partly achieved by a method for producing a liquid oat base for human consumption, a method for producing a liquid oat product for human consumption, and a corresponding system, as defined by the independent claims, with further embodiments specified in the dependent claims.

The present disclosure is based on the surprising finding by the Applicant that the amount of residue in the production of a liquid oat base from oat kernels or whole oat flour can be significantly reduced, while still obtaining product with a desirable mouthfeel, by a two-step homogenization process. By reducing the amount of residue, the environmental impact and disposal costs of the production process are minimized, as less waste needs to be handled. Furthermore, a larger fraction of the oat raw material may be retained in the liquid oat base, leading to an increased yield and potentially higher nutritional value in the final product.

The Applicant has conducted extensive research and systematic trials, testing multiple different production methods to evaluate their efficiency and end-product quality. While many of these methods produced similar results in some aspects, it was discovered that the total production cost varied between them. Although the cost differences were relatively small when measured in percentage terms, the cumulative effect on resource consumption and long-term sustainability was found to be substantial. Over time, even minor improvements in process efficiency translate into significant savings in energy and raw material utilization, making the optimized process highly advantageous.

Additionally, it was observed that the final characteristics of the liquid oat base, in some embodiments and in addition to the two-step homogenization, are influenced by the specific pre-processing used to create the oat-water slurry. In particular, variations in grinding techniques and milling conditions prior to homogenization were found to impact factors such as not only texture, stability, and mouthfeel of the liquid oat base but also the overall resource cost. Ensuring an optimized pre-processing step is therefore critical for achieving a smooth, non-gritty texture while maintaining high production efficiency.

Still other objectives, technical advantages, aspects, features, and embodiments will become apparent from the following detailed description and accompanying drawings.

### Drawings

Fig. 1 is a flow chart illustrating a method for producing a liquid oat base and a method for producing a liquid oat product.
Fig. 2 illustrates a system for producing a liquid oat base.
Fig. 3 illustrates a system for producing a liquid oat product.
Fig. 4 illustrates a sub-system configured to process oat raw material, water, and one or more enzymes into a slurry.
Fig. 5 illustrates a control device arranged to regulate a system for producing a liquid oat base and/or a liquid oat product.
Fig. 6 is a diagram illustrating the relationship between product particle size and homogenizer power requirements for producing a liquid oat base, as a function of pressure.
Fig. 7 is a column chart illustrating a cost comparison of different system setups used for producing a liquid oat product.

### Description

Embodiments will now be described in greater detail with reference to the accompanying drawings, which illustrate some, but not all, embodiments. Indeed, the subject of the present disclosure may be embodied in many different forms and should not be construed as limited to the embodiments set forth herein.

Well-known functions or constructions may not be described in detail for brevity and/or clarity. Unless otherwise defined, all terms (including technical and scientific terms) used herein have the same meaning as commonly understood by one of ordinary skill in the art to which this invention belongs. Like reference signs refer to like elements throughout.

Before describing embodiments in more detail, a few definitions will be given.

As used herein, "oat raw material" refers to oat-derived material used as the starting ingredient for producing a liquid oat base. The oat raw material may comprise either "oat kernels" or "whole oat flour", or a combination thereof.

As used herein, "oat kernels" refer to de-hulled oats (*Avena sativa*), also known as oat groats, which may have any size or shape. For example, oat kernels may be whole, cut, flaked, or otherwise processed while still retaining all parts of the oat except the hull.

As used herein, "whole oat flour" refers to flour produced from de-hulled oat kernels (oat groats), meaning it contains all components of the oat except the hull. In some embodiments, whole oat flour has a particle size (D90, dry) in the range of 500-800 µm.

As used herein, a "liquid oat base" or "oat base" is a liquid that serves as a starting material for further formulation into oat beverages or other oat-based products for human consumption. The liquid oat base may be considered a "precursor liquid" for a final oat-based product. In some embodiments, the liquid oat base is a suspension. Typically, the liquid oat base lacks one or more properties that make it suitable for direct human consumption, such as the desired taste, texture and/or ingredients.

As used herein, a "slurry" refers to a mixture of solid particles suspended in a liquid phase, where the solids are denser than the liquid.

As used herein, "solid content " refers to the solid content of the oat raw material present in a sample, including suspended, colloidal, and dissolved solids. The solid content is typically determined as the amount of solid material remaining after heating the sample at 105°C to constant weight. In some embodiments, the total solids content is expressed as a percentage of the total weight of the sample, corresponding to the solid content of the oat raw material in the production process.

The term "D90" is used in its ordinary meaning and refers to a measure of particle size, where 90% of the particles in a given mixture have a diameter smaller than the D90 value. In some embodiments, particle size is measured using laser diffraction techniques.

As used herein, "high-pressure homogenization" refers to a process in which a fluid is forced under high pressure through a narrow slot or gap, resulting in particle breakdown due to cavitation, shearing, and collisions. Conventionally, the pressure ranges from 40 to 2000 bar.

A "high-pressure homogenizer" is a device configured to perform high-pressure homogenization. It typically includes a mechanical structure that defines the gap, which may be adjustable, and a pumping mechanism that generates the pressure to drive the fluid through the gap.

As used herein, the terms "disc mill" is used in its ordinary meaning and refers to a milling device that grinds, shears, or crushes material using one or more rotating discs. The discs may have grooved, serrated, or perforated surfaces, and the gap between them may be adjustable to control particle size.

As used herein, the term "colloid mill" is used in its ordinary meaning and refers to a milling device that reduces particle size in liquid or semi-liquid materials by applying shear, impact, and turbulence between a rotating cone (rotor) and a stationary cone (stator). The gap between the rotor and stator may be adjustable to control the degree of milling.

As used herein, "wet milling" refers to milling process in which solid materials (oat raw material) are ground, dispersed, or reduced in size while simultaneously suspended or combined with water.

As used herein, a "high-shear mixing" refers to a process that involves a mixing unit, for example in form of a rotor, impeller, paddle, propeller, vane, turbine etc., that rotates with a peripheral speed of at least 10 m/s, at least 15 m/s, or at least 20 m/s, to hit particles and thereby reduce their size of particles by breaking them up.

Any methods described herein may be computer-implemented and performed by a control device configured to regulate equipment that executes the steps of the method. The control device may be implemented in hardware or a combination of software and hardware.

Any methods described herein may be computer-implemented and performed by a control device configured to regulate equipment that executes the steps of the method. The control device may be implemented in hardware or a combination of software and hardware. For example, the control device may comprise processor circuitry, computer memory, and a signal interface. A control program containing computer instructions may be stored in the computer memory and executed by the processor circuitry to perform methods and procedures as described herein, by generating and outputting control signals via the signal interface. The control program may regulate and/or control processing equipment, such as homogenizers or mills, to perform the different steps of the method.

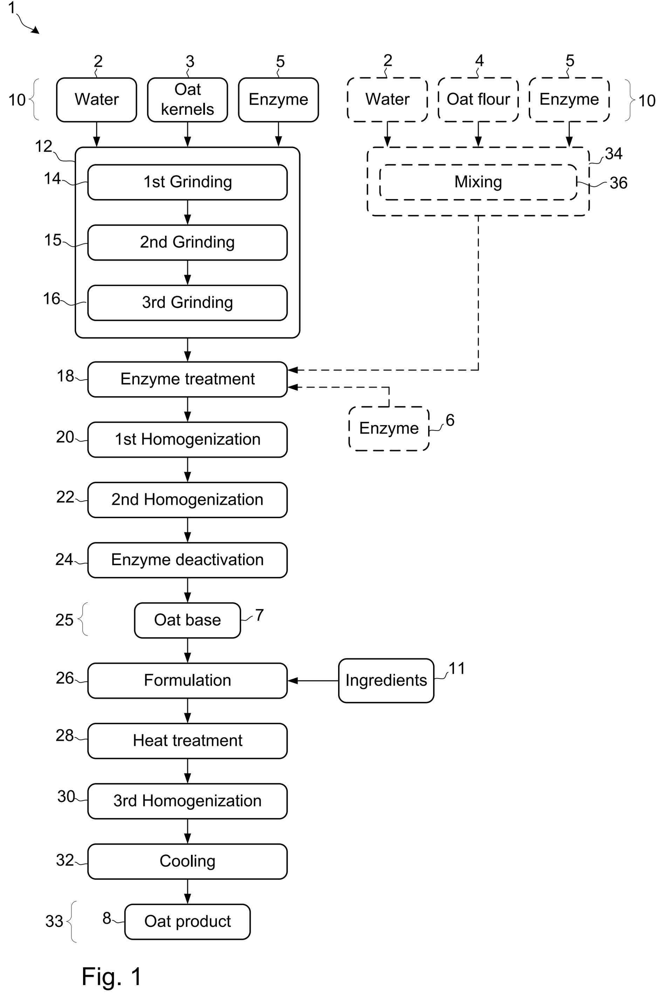

Fig. 1 is a flow chart illustrating a method 1 for producing a liquid oat base 7 and a liquid oat product 8. The method begins with providing, or inputting 10, oat raw material and water 2. The oat raw material may be oat kernels 3. One or more enzymes 5 are also introduced to the oat kernels 3 and water, either from the onset or at some time after the kernels and water have been combined.

The oat kernels 3 and the water 2 are processed 12 into a slurry, which includes a grinding sequence to reduce the particle size of the oat kernels. The grinding sequence comprises a first grinding step 14, a second grinding step 15, and an optional third grinding step 16. These steps are designed to break down the oat kernels into fine particles and produce a uniform slurry that is ready for subsequent processing steps.

Specifically, in the first grinding step 14, the oat raw material undergoes wet milling in a disc mill. In this step, the oat raw material 3 and water 2 are fed into the disc mill, where rotating discs with grooved or serrated surfaces grind the material into a coarse slurry. An adjustable gap between the discs may allow for control over the particle size, ensuring the material is ground to the desired particle size. This process effectively shears and crushes the oats, facilitating hydration and enzyme interaction in subsequent steps.

In the second grinding step 15 the slurry from the first grinding step 14 is processed in a colloid mill. Here, the slurry passes through a narrow gap between a high-speed rotating rotor and a stationary stator, both of which may have adjustable spacing to control the degree of milling. The intense shear forces, impact, and turbulence within the colloid mill further reduce the particle size, resulting in a finer slurry with improved homogeneity. This step enhances the breakdown of oat particles, promoting a smoother texture in the final product.

In the third grinding step 16 the slurry undergoes further and final grinding, using an additional colloid mill. This step is employed to achieve an even finer particle. By subjecting the slurry to further shear and impact forces, this step ensures a highly uniform and stable slurry, optimizing it for subsequent processing stages such as homogenization.

Essentially, the first grinding step 14 grinds oat raw material 3 and the water 2 into a one-step ground slurry, the second grinding step 15 grinds the one-step ground slurry into a two-step ground slurry, and the third grinding step 16 grinds the two-step ground slurry into a three-step ground slurry.

Following the production of the slurry in step 12, an enzyme treatment step 18 is performed. In this step, the enzymes 5 hydrolyze carbohydrates in the slurry into simpler sugar molecules. The enzymes 5 can be combined with the oat raw material and the water at any suitable time. The enzyme treatment 18 is crucial for breaking down starches and enhancing the digestibility and texture of the final oat base. Depending on the process configuration, additional enzymes 6 may also be introduced to further refine the slurry composition.

In the context of the present disclosure, the enzyme treatment is also denoted "hydrolysis" or "hydrolyzation". Hydrolysis is a process in which a molecule of water breaks one or more chemical bonds. The enzymatic treatment comprises adding the one or more enzymes to the slurry and allowing the respective enzyme to break down components of the slurry for a predefined hydrolyzation time period. It is currently believed that an effective enzymatic treatment is achieved by adding the respective enzyme in an amount of 0.5%-3% by weight of the slurry. Control parameters of the enzymatic treatment, such as temperature, pH, and treatment time, are adapted to the respective enzyme. Step 18 results in a saccharification of the slurry.

In some embodiments, the enzyme(s) used in step 18 comprises one or more carbohydrases that cause saccharification of starch by hydrolysis. Successful tests have been performed by adding an alfa-amylase and/or a glucoamylase to the slurry. In the tests, BAN^{®} 440 L from Novozymes was used as alfa-amylase, and AMG^{®} 300 L from Novozymes was used as glucoamylase. It is to be understood that any suitable, commercially available alfa-amylase and/or glucoamylase may be added to slurry.

The enzyme-treated slurry is then subjected to a two-step high-pressure homogenization process, which includes a first homogenization step 20 and a second homogenization step 22. These steps are performed in series to achieve a uniform particle size distribution and ensure stability of the liquid oat base. The two-step homogenization process is critical for reducing particle size through cavitation, shear forces, and collisions, resulting in a smoother texture in the final product. Each of the homogenization steps 20, 22 may comprise high-pressure homogenization.

Following the second homogenization step 22, an enzyme deactivation step 24 is carried out to terminate enzyme activity. This step typically involves heat treatment, where the slurry is exposed to a predefined temperature for a specific duration to deactivate the enzymes and stabilize the liquid oat base. In some embodiments, the enzyme deactivation step 24 may occur prior to the first homogenization step 20, or between the first and second homogenization steps 20, 22.

The processed liquid is then identified and provided 25 as the liquid oat base 7, which may serve as a precursor for producing a liquid oat product. In subsequent processing, steps are performed to produce a liquid oat product 8 from the liquid oat base 7. The liquid oat base 7 then undergoes formulation 26, where additional ingredients 11, such as one or more of vegetable oil, flavoring, sweetener, salt, thickener, stabilizer, vitamin, or mineral, are mixed into the liquid oat base 7. Heat treatment 28, such as pasteurization or UHT (ultra-high-temperature) treatment is may be performed to pasteurize or sterilize the formulated product. A third homogenization step 30 may follow the heat treatment to ensure uniform distribution of added ingredients and further improve the product's texture. The third homogenization step 30 may comprise high-pressure homogenization.

The final steps of the process include cooling 32, which reduces the temperature of the liquid oat product to storage or packaging conditions. In the final step 33, the processed liquid is obtained as the liquid oat product 8. The liquid oat product 8 may, for example, be a beverage, a yoghurt substitute, a cream substitute, a custard, a sour cream or soured cream substitute, or a raw material to produce oat based cheese or an ice cream. The method may further comprises a filling step (not shown), in which the oat product 8 product from step 33 is filled and enclosed in a suitable package for distribution, including but not limited to bottles of glass or plastic, metal cans, or carton-based packages.

Certain steps may vary in order or implementation. For example, the heat treatment 28 and third homogenization step 30 may be performed in reverse order. This adaptability allows for tailoring the process to specific production requirements or product characteristics.

An alternative method for processing oat raw material involves processing step 34, in which oat flour 4 is combined with water 2 and one or more enzymes 5. As part of step 34, high-shear mixing 36 may be performed to create a homogeneous slurry with a desired particle size distribution. The high-shear mixing process ensures that the enzymes are evenly distributed throughout the slurry, enabling efficient hydrolyzation in subsequent steps. This method provides flexibility by eliminating the need for grinding steps 14, 15, and 16, since oat flour 4 is already in a fine particulate form. The slurry resulting from the high-shear mixing 36 in processing step 34 is then subjected to the same enzyme treatment 18 and subsequent steps as the slurry that was prepared by the processing steps 12 that included the three grinding steps 14, 15, 16.

The method depicted in Fig. 1 highlights the efficient utilization of oat raw material, reducing residue and optimizing resource use. The two-step homogenization and careful control of particle size ensure that the final product achieves a smooth mouthfeel, stability, and high-quality texture, suitable for human consumption.

In summary, in its general form the method is used for producing a liquid oat base 7 for human consumption and includes processing 12 or 34 oat raw material, water 2, and one or more enzymes 5 into a slurry. The method further involves hydrolyzing 18 the slurry over a period of time using the one or more enzymes 5. The hydrolyzed slurry is then homogenized 20 by high-pressure homogenization in a first homogenization step, followed by homogenizing 22 the homogenized slurry by high-pressure homogenization in a second homogenization step. The one or more enzymes are subsequently deactivated 24 in the two-step homogenized slurry, and the liquid oat base 7 is obtained 33 from the two-step homogenized slurry.

The optimized method provides significant advantages in terms of efficiency and sustainability. As will be described further on, the improvement in production cost, though minor in percentage, results in substantial resource and energy savings over time, enhancing long-term sustainability. The specific pre-processing techniques, including the two-step homogenization, and, in one embodiment the grinding and milling conditions, play a crucial role in achieving a smooth, stable, and high-quality liquid oat base while maintaining cost efficiency. The method ensures improved texture and mouthfeel while optimizing raw material utilization.

In one embodiment, the slurry is produced to include at least 90%, 95%, or 98% by weight of a solid content of the oat raw material 3. This high solid content enhances resource utilization by ensuring that the majority of the raw material, including fibers, contributes to the final product.

In one embodiment, the two-step homogenized slurry has a particle size, given as D90, of less than 200 µm, or less than 150 µm. This fine particle size results in a smoother texture and more stable liquid oat base 7, improving the quality of the final product.

In one embodiment, the two-step homogenized slurry has a particle size, given as D90, of at least 100 µm, or at least 110 µm. Maintaining this particle size prevents over-processing, reducing viscosity issues and ensuring optimal consistency for further use.

In one embodiment, the slurry is processed in the first homogenization step by high-pressure homogenization at a pressure of at least 100 bar, at least 200 bar, or at least 400 bar. This high pressure ensures effective particle breakdown, resulting in a more uniform slurry.

In one embodiment, the slurry is processed in the first homogenization step by high-pressure homogenization at a pressure of at most 700 bar, or at most 500 bar. Limiting the pressure reduces energy consumption and prevents excessive mechanical stress on the slurry.

In one embodiment, the homogenized slurry is processed in the second homogenization step by high-pressure homogenization at a pressure of at least 300 bar, at least 500 bar, or at least 700 bar. This ensures further refinement of the slurry to achieve a smooth and stable liquid oat base 7.

In one embodiment, the homogenized slurry is processed in the second homogenization step by high-pressure homogenization at a pressure of at most 1000 bar, or at most 800 bar. This controlled pressure prevents over-processing and ensures efficient energy use.

In one embodiment, each of the high-pressure homogenization steps includes driving the respective slurry through a homogenization gap with a size of 50 to 250 µm. This precise gap size facilitates consistent particle reduction, ensuring a stable and high-quality oat base.

In one embodiment, the processing of oat raw material 3, water 2, and one or more enzymes 5 into a slurry includes grinding, in a first grinding step 14, the oat raw material 3 and water 2 with a disc mill into a one-step ground slurry. This step ensures initial particle size reduction and hydration of the oat raw material.

In one embodiment, the processing includes grinding, in a second grinding step 15, the one-step ground slurry with a colloid mill into a two-step ground slurry. This provides finer particle size reduction and improved uniformity of the slurry.

In one embodiment, the processing includes grinding, in a third grinding step 16, the two-step ground slurry with a further colloid mill into a three-step ground slurry. This optional step ensures even finer particle size reduction, improving the texture and stability of the liquid oat base 7.

In one embodiment, no other grinding steps than the three described grinding steps 14, 15, 16 are performed on the slurry prior to subjecting it to homogenization.

In one embodiment, no other high-pressure homogenization steps are performed apart from the first homogenization step 20 and the second homogenization step 22, which are carried out in series with no product-altering step in between. This ensures an efficient and streamlined process with minimal complexity.

As used herein, "product-altering step" may refer to the product, or slurry, being affected by equipment that alters it shape, form, content, particle distribution, or transfers heat to or from the product or slurry.

In one embodiment, a liquid oat product 8 is produced by formulating the liquid oat base 7 with one or more ingredients 11, followed by high-pressure homogenization in a third homogenization step 30. This formulation step allows for customization of the final product while maintaining its smooth texture and stability.

An embodiment that combines two-step homogenization and three-step milling provides a cost-efficient yet high-quality production process for a liquid oat base. The three-step milling process, which progressively refines particle size, ensures efficient raw material utilization and minimizes waste, reducing unnecessary by-product disposal costs. Meanwhile, the two-step high-pressure homogenization process optimizes particle uniformity and texture without excessive energy consumption, achieving a smooth and stable liquid oat base. Together, these processes balance low total production cost with a high-quality end product, ensuring an attractive mouthfeel while maintaining efficient use of resources and energy.

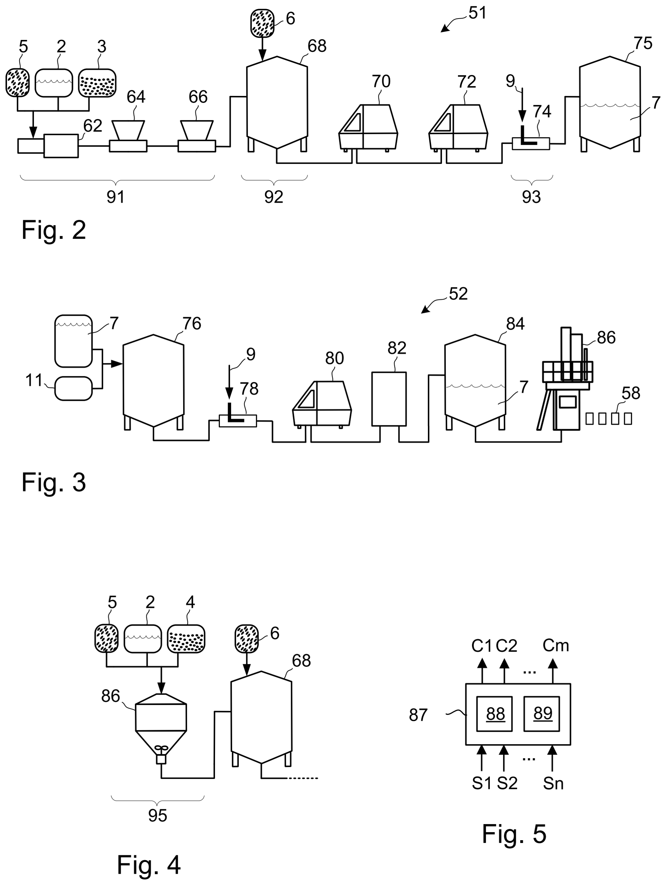

Fig. 2 illustrates a system 51 for producing a liquid oat base 7 for human consumption. The system 51 is arranged to perform the method (process) up until the step 25 of providing the liquid oat base 7, described in Fig. 1. The system 51 comprises three interconnected sub-systems; a first sub-system 91, a second sub-system 92, and a third sub-system 93 which together with two homogenizers 70, 72 perform specific stages of the production process. The arrangement and interaction of these sub-systems ensure efficient processing of oat raw material, water 2, and one or more enzymes 5 into the liquid oat base 7. One or both of the two homogenizers 70, 72 may be high-pressure homogenizers.

The process begins in the first sub-system 91, which is configured to process oat kernels 3, water 2, and one or more enzymes 5 into a slurry. The first sub-system 91 includes a disc mill 62, which performs initial grinding of the oat kernels 3 and water 2 to form a one-step ground slurry. The disc mill 62 applies shear and cutting forces to the oat kernels 3, reducing their particle size while ensuring thorough mixing with water 2. The slurry is then transferred to the first colloid mill 64, which further grinds the slurry to reduce the particle size and produce a two-step ground slurry. For further refinement, the two-step ground slurry is processed in the second colloid mill 66, which results in a three-step ground slurry with finely dispersed particles. This three-step grinding process ensures that the slurry has an optimal particle size distribution, achieving a smooth and stable liquid oat base 7.

The ground slurry is then transferred to the second sub-system 92, which includes a tank 68 (or multiple tanks) designed for hydrolysis, also referred to as enzymation. In the tank 68, one or more enzymes 6 may be added to the slurry. The enzymes 6 break down oat starch into simpler sugars over a controlled time period, ensuring that the slurry develops the desired nutritional and textural properties. The tank 68 is configured to allow precise control over enzymatic reactions, ensuring consistency and quality in the production process.

The enzymatically treated slurry is then pumped from the second sub-system 92 to the first homogenizer 70, where it undergoes high-pressure homogenization in the first homogenization step 20. The first homogenizer 70 applies pressure to break down larger particles, ensuring uniformity and stability in the slurry. The partially homogenized slurry is then transferred to the second homogenizer 72, where the second high-pressure homogenization step 22 is performed. The second homogenizer 72 applies even higher pressure to achieve a finer particle size distribution, resulting in a smooth and stable slurry.

Following homogenization, the two-step homogenized slurry is directed to the third sub-system 93, which is configured to deactivate the enzymes 6. For this purpose, the third sub-system 93 may comprise a steam injector 74, where steam 9 is injected into the slurry to deactivate the enzymes 6, stabilizing the slurry and thereby forming the liquid oat base 7 by halting any further enzymatic reactions.

The stabilized liquid oat base 7 is transferred to a storage tank 75, where it is collected and stored for subsequent formulation or packaging. The storage tank 75 ensures that the liquid oat base 7 is maintained under controlled conditions, ready for further use.

The system 51 integrates the functionalities of grinding, hydrolysis, homogenization, and enzyme deactivation into a streamlined and efficient process. The combination of the disc mill 62, first colloid mill 64, and second colloid mill 66 in the first sub-system 91 ensures thorough particle size reduction and uniformity in the slurry. The hydrolysis process in the tank 68 of the second sub-system 92 facilitates enzymatic conversion, while the first homogenizer 70 and second homogenizer 72 between the second sub-system 92 and the third sub-system 93 deliver the required particle size and stability. Finally, the steam injector 74 in the third sub-system 93 effectively deactivates the enzymes 6, halting enzymatic activity and producing the liquid oat base 7.

Fig. 3 illustrates a system 52 for producing a liquid oat product 8 for human consumption. The system 52 is arranged to perform the method (process) steps after the step 25 of providing the liquid oat base 7, described in Fig. 1. The system 52 begins by receiving the liquid oat base 7, produced in system 51 (as shown in Fig. 2), as its primary input. Additional ingredients 11, such as flavorings, sweeteners, or stabilizers, are also introduced to create a formulated mixture.

The liquid oat base 7 and ingredients 11 are fed into a mixing tank 73, where they are combined to form a pre-formulated oat mixture. The mixing tank 73 ensures that the ingredients 11 are uniformly distributed within the liquid oat base 7.

The pre-formulated oat mixture is then transferred to the UHT unit 78, which can be a steam injector, where steam 9 is injected to heat-treat the mixture, raising its temperature to a sterilization level suitable for microbial inactivation. This step prepares the mixture for subsequent homogenization while ensuring that microbial contaminants are eliminated. Any suitable, conventional temperature and time values may be used for performing the UHT.

After heat treatment 28, the mixture is directed to the third homogenizer 80, where it undergoes high-pressure homogenization in step 30.The third homogenizer 80 may be a high-pressure homogenizer. The third homogenizer 80 is critical for reducing the particle size of the mixture and performing final mixing, resulting in a smooth and uniform consistency in the liquid oat product 8. This step enhances the texture and mouthfeel of the product while ensuring its stability.

The homogenized mixture is then transferred to a cooler 82, where it is cooled to a controlled temperature. The cooler 82 prevents thermal degradation of the product, ensuring that its quality and shelf life are preserved.

Once cooled, the liquid oat product 8 is stored in a storage tank 84. The storage tank 84 provides a controlled environment to maintain the product's quality and stability before it is packaged. From the storage tank 84, the liquid oat product 8 is fed into a packaging machine 86. The packaging machine 86 aseptically fills the liquid oat product 8 into individual packages 58. These packages 58 are designed to maintain sterility, product stability, and readiness for distribution.

The system 52 integrates the functionalities of formulation, UHT treatment, homogenization, cooling, and packaging to produce a high-quality liquid oat product 8. By combining the liquid oat base 7 from system 51 with ingredients 11 in the mixing tank 73, the system 52 enables the production of a wide range of oat-based products tailored to consumer preferences.

Fig. 4 illustrates an alternative sub-system 95 that can be used in place of sub-system 91 when the oat raw material is whole oat flour 4 instead of oat kernels 3. This sub-system 95 is configured to process whole oat flour 4, water 2, and one or more enzymes 5 into a slurry, preparing it for enzymatic treatment.

The process begins with whole oat flour 4 and water 2 being introduced into the sub-system 95, which comprises a high-shear shear mixing unit 86. The components are thoroughly mixed and dispersed in the mixing unit 86 to form a slurry. The mixing unit 86 ensures that the whole oat flour 4 is evenly distributed in water 2, forming a homogeneous slurry suitable for enzymatic hydrolysis.

The slurry from the mixing unit 86 is then transferred to a tank 68, where enzymatic treatment occurs. Further enzymes 6 are introduced into the slurry in tank 68 to hydrolyze oat starches into simpler sugars. The enzymatic treatment process in tank 68 is precisely controlled for time and temperature to ensure optimal starch hydrolysis, which is critical for subsequent processing.

This alternative sub-system 95 is specifically adapted for use with whole oat flour 4, providing flexibility in oat raw material selection while ensuring compatibility with downstream processing steps in system 51. The tank 68 in Fig. 4 serves the same purpose as tank 68 in Fig. 2, facilitating precise and controlled enzymatic treatment of the slurry. Therefore, after enzymatic treatment in tank 68, the process continues as described for system 51 in Fig. 2.

Fig. 5 illustrates a control device 87 configured to regulate and control the equipment used in the systems for producing a liquid oat base and liquid oat product, as described in previous figures. The control device 87 facilitates the automation and optimization of various process steps by receiving sensor inputs and generating control signals to manage system operations.

The control device 87 comprises a processor 88 and a memory 89. The processor 88 is configured to execute control programs stored in the memory 89. These control programs contain instructions for performing the control tasks associated with the processes illustrated in the earlier figures, i.e. for performing the various method steps described in connection with Fig. 1. For instance, the processor 88 can regulate grinding speeds, enzyme dosing, homogenization pressures, or UHT conditions based on the operational requirements of the system.

The control device 87 receives multiple sensor inputs S1, S2, ... Sn from the equipment in the systems. These sensors may monitor parameters such as temperature, pressure, flow rate, particle size, or enzyme activity. The sensor inputs enable the processor 88 to adjust process parameters dynamically and maintain optimal operating conditions throughout the production cycle.

The control device 87 generates control signals C1, C2, ... Cm, which are sent to various actuators or components in the system. These control signals ensure that equipment such as mills, homogenizers, UHT units, and mixing tanks operate according to the desired settings, such that the method described herein can be performed.

Water, slurry, liquid oat base, and liquid oat product are transferred between the components and equipment in the systems 51, 52 using conventional pumps and valves. Pumps ensure consistent flow and pressure, while valves regulate and direct the material flow, enabling efficient and seamless operation throughout the production process.

The system may employ conventional milling and homogenization equipment of the types described herein to achieve optimal particle size and consistency. For milling, devices such as perforated disc mills and colloid mills are used, ensuring effective particle size reduction and slurry uniformity. Homogenization may be performed using a Tetra Pak^{®} homogenizer equipped with an HD 100 homogenizing device, ensuring a smooth and stable final product.

Examples of enzymes that can be used include glucanases, such as Ultraflo^{®} XL from Novozymes, and xylanases, such as Ultraflo^{®} Max from Novozymes. These enzymes are effective for breaking down fibers like beta-glucans and xylans in the slurry, either in combination with starch-saccharifying enzymes or separately.

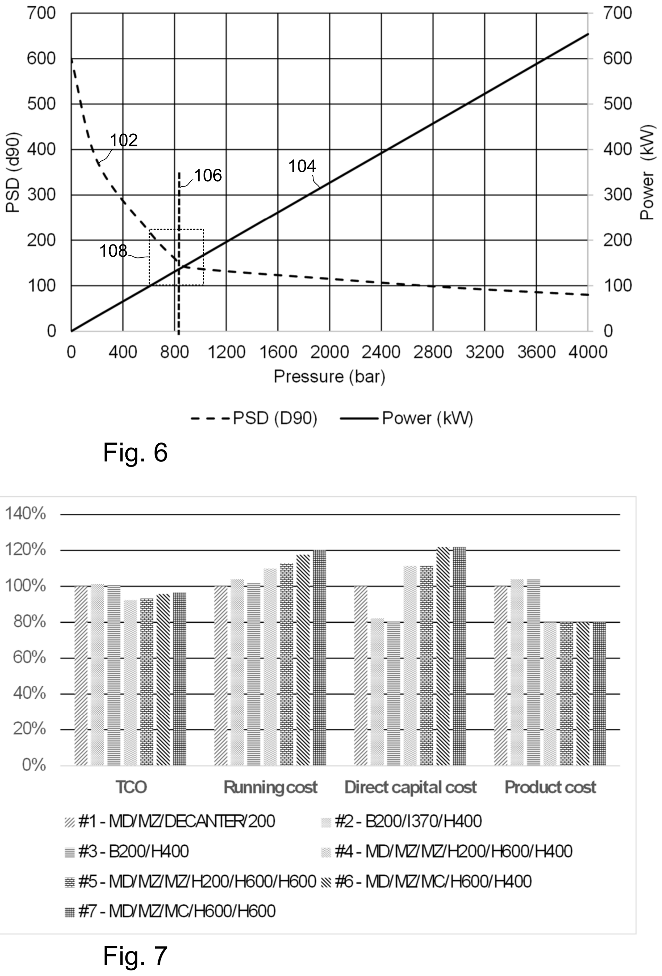

Fig. 6 illustrates how low total production cost and a high-quality end product can be balanced, showing the relationship between pressure in bar, particle size distribution (PSD) measured as D90 in µm, and power requirements measured in kW for the second homogenization step 22 in the method 1.

The dashed line, labeled 102, represents the particle size distribution (PSD), measured in terms of D90 (µm), where 90% of the particles in the mixture have a diameter smaller than the D90 value. As the pressure increases, the PSD decreases, indicating a finer particle size in the slurry.

The solid line, labeled 104, represents the total power requirement in kW for a homogenizer used for the second homogenization step 22. The power requirement increases linearly with pressure, reflecting the energy needed to achieve finer particle sizes.

The vertical dashed line, labeled 106, marks a particularly optimal pressure, having a value of about 850 bar. At this pressure, the PSD is sufficiently low to produce a smooth and uniform liquid oat base, while the power consumption remains reasonable, maximizing energy efficiency.

The box, labeled 108, indicates a range of pressures that achieves a balance between a good PSD (resulting in a high-quality product) and moderate energy consumption, ensuring resources are not overused. This range, which is from about 600 to 1000 bar, gives a good particle size for a smooth texture and stable liquid oat base while maintaining cost-efficiency.

Fig. 6 highlights the importance of selecting an appropriate homogenization pressure to balance product quality and energy usage, ensuring the process remains both sustainable and cost-effective.

The slurry that was prepared had the following relative composition: Oat 1778 kg/h (of which 1600 kg/h is dry oat), enzyme 5,0 kg/h, water 6222 kg/h. The total weight is then 8000 kg/h and the dry matter constituted 20% of the total weight, not taking the weight of the enzyme into account. The same composition was used for tests performed in seven different systems set ups, as described below.

Fig. 7 is a column chart comparing the cost components for seven different system setups used for producing a liquid oat product. The setups, labeled #1 to #7, represent different configurations of equipment and process conditions, highlighting their impact on total cost of ownership (TCO), running costs, direct capital costs, and product costs. In Fig 7, the costs of setup #1 is used as the references for normalization, representing 100%.

Setup #1, labeled MD/MZ/DECANTER/200, represents a traditional oat beverage production line where oat fibers are removed. It includes a perforated disc mill MD for initial grinding, a colloid mill MZ for further size reduction, enzyme treatment, deactivation, a decanter for fiber separation, formulation, ultra-high temperature (UHT) treatment, a homogenizer running at 200 bar, and an aseptic tank.

Setup #2, labeled B200/I370/H400, starts from oat flour, eliminating the need for grinding. It incorporates a high-shear mixer B200 and an in-line high-shear mixer I370 for mixing and homogenization. The system includes UHT treatment with a homogenizer running at 400 bar for the formulated product. No decanter is used, as the flour-based process avoids fiber separation.

Setup #3, labeled B200/H400, is similar to setup #2 but does not include the in-line high-shear mixer I370. It features a high-shear mixer B200 and UHT treatment with a homogenizer running at 400 bar.

Setup #4, labeled MD/MZ/MZ/H200/H600/H400, is the solution described in this patent application. It includes a perforated disc mill MD and two colloid mills MZ for grinding and size reduction. Two homogenizers are used: the first running at 200 bar and the second at 600 bar for breaking up fibers in the liquid oat base. A third homogenizer, running at 400 bar, is used for the formulated product.

Setup #5, labeled MD/MZ/MZ/H200/H600/H600, is similar to setup #4 but uses a third homogenizer running at 600 bar instead of 400 bar for the formulated product.

Setup #6, labeled MD/MZ/MC/H600/H400, features a perforated disc mill MD, a colloid mill MZ, and a microcut unit MC for grinding and size reduction. A single homogenizer running at 600 bar is used for breaking up fibers in the liquid oat base, followed by a second homogenizer at 400 bar for the formulated product.

Setup #7, labeled MD/MZ/MC/H600/H600, is similar to setup #6 but uses a second homogenizer running at 600 bar instead of 400 bar for the formulated product.

All setups included further equipment, e.g., for hydrolyzing, formulating and heat treating the product. Such further equipment were same for all setups.

Among the seven setups, setup #4 is identified as the most efficient and cost-effective solution, balancing low total production cost with high-quality output, as evidenced by Fig. 7. Setup #4 achieves one of the lowest TCO values, reflecting its cost-effectiveness over the system's lifecycle. Its running costs are lower than those of setups with higher-pressure homogenizers for breaking up fibers, such as setups #5, #6, and #7, ensuring efficient energy use. Setup #4 also avoids the high capital costs associated with setups that incorporate more complex components, such as the microcut unit in setups #6 and #7.

The product cost for setup #4 is among the lowest by integrating oat fibers into the final product, avoiding the need for fiber separation, which reduces waste while maintaining high product quality. Tests have shown that setup #4 employs an optimal combination of homogenization pressures, 200 bar and 600 bar for the liquid oat base, and 400 bar for the formulated product. This ensures effective particle size reduction, resulting in a smooth and uniform liquid oat base without overusing energy resources. Setup #4 reflects the innovative method and system described in this patent application, combining a perforated disc mill and two colloid mills with an optimized three-stage homogenization process. This setup achieves good product quality while minimizing costs and resource consumption.

In addition to the embodiments described above, further embodiments and variants are described in the following. Unless explicitly stated otherwise, features of the different embodiments described herein may be combined with each other in any technically reasonable manner.

In some additional embodiments, the processing of oat raw material and water into a slurry includes a multi-stage wet-milling sequence having at least two, three, four or five successive grinding stages, wherein each stage may employ a different type of mill, a different tool geometry and different operating parameters. A method for producing a slurry may therefore comprise performing successive grinding stages in mills of different construction and function to obtain a targeted particle-size distribution.

In one group of embodiments, the oat raw material and water are first coarsely ground in a disc mill to form a coarse slurry, after which the slurry is processed in one or more colloid-mill stages to obtain a fine slurry. The disc mill may comprise one or more rotating discs provided with grooves or serrations, wherein the groove profile may be straight, helical, chevron-shaped or sinusoidal. The disc gap may be adjustable in the range of 50 to 1500 micrometres, optionally in the range of 100 to 800 micrometres or 200 to 600 micrometres. A process may thus comprise adjusting a disc-mill gap within these ranges while grinding oat raw material with water.

In a further group of embodiments, the disc mill is operated at a controllable rotational speed, for example between 300 and 3000 revolutions per minute, such that the specific grinding energy input is controlled based on one or more measured parameters including torque, power consumption, product temperature and slurry viscosity. The disc mill may include temperature-control means, including a jacketed housing with a heat-transfer fluid, configured to maintain the slurry at a temperature within a target range during grinding, for example 20 to 65 degrees Celsius, 30 to 55 degrees Celsius or 40 to 50 degrees Celsius. A method may therefore include regulating the disc-mill temperature during grinding so as to maintain the slurry within a predetermined temperature window.

In subsequent grinding stages, the slurry may be processed in one or more colloid mills having a rotor-stator configuration. The rotor-stator gap may be fixed or dynamically adjustable during operation, for example between 10 and 500 micrometres, preferably between 20 and 300 micrometres. The rotor-stator surfaces may be smooth, grooved or provided with teeth. Different colloid-mill stages may have different gap sizes, surface textures and rotational speeds in order to tailor the particle-size distribution of the slurry. A method may therefore comprise dynamically adjusting a rotor-stator gap in the course of producing the slurry.

In certain embodiments, a three-step wet-milling sequence is used in which a first grinding step is performed in a disc mill to provide a one-step ground slurry, a second grinding step is performed in a first colloid mill to provide a two-step ground slurry and a third grinding step is performed in a second colloid mill to provide a three-step ground slurry, wherein the first and second colloid mills have different rotor-stator geometries, different gap sizes and different rotational speeds. Such an embodiment may be expressed as a method comprising consecutively grinding a slurry in a disc mill, a first colloid mill and a second colloid mill, each configured with distinct operational parameters.

In some embodiments, inline particle-size measurement is performed during one or more grinding steps. The slurry may be circulated through a measurement cell of a laser-diffraction instrument, a dynamic light-scattering device, an image-analysis system, an ultrasonic sensor, an acoustic sensor or any combination of these. The measured particle-size distribution, including values such as D10, D50 and D90, may be fed to a control device which automatically adjusts at least one grinding parameter including disc-mill gap, rotor speed, feed rate or slurry temperature to maintain the particle-size distribution within a desired range. Accordingly, a process may comprise automatically modifying grinding parameters based on inline measurement signals.

In particular embodiments, the grinding sequence is controlled such that the ground slurry exiting the final milling stage has a D90 in the range of 80 to 250 micrometres, preferably 90 to 200 micrometres and more preferably 100 to 160 micrometres. The D50 may be in the range of 20 to 120 micrometres, for example 30 to 90 micrometres. The D10 may be in the range of 2 to 50 micrometres, for example 5 to 30 micrometres. The particle-size distribution may have a ratio of D90 to D10 between 2 and 10, for example between 3 and 7, which indicates a relatively narrow distribution. A method may therefore comprise controlling grinding conditions such that the slurry exiting a final milling stage meets at least one of these particle-size criteria.

In some embodiments, the milling sequence is configured such that at least 90 weight percent, 95 weight percent, 97 weight percent or 99 weight percent of the solids of the oat raw material are retained in the slurry, thereby reducing removal of coarse waste. This may be achieved by appropriate selection of the mill types, gaps, speeds and recirculation flows, optionally combined with mild classification steps which remove only exceptionally large particles. A method for producing a slurry may therefore comprise retaining at least 90 percent of the solids of the oat raw material throughout the grinding sequence.

In further embodiments, the wet-milling sequence includes four or five grinding stages. These may include disc mill, colloid mill, colloid mill, high-shear mixer and colloid mill in succession, or disc mill, disc mill, colloid mill and colloid mill in succession. At least one of the stages may be a high-shear mixing stage in which the slurry is subjected to rotor-stator mixing at a tip speed of at least 10 metres per second, 15 metres per second or 20 metres per second, thereby reducing agglomerates and improving slurry homogeneity. A process may therefore comprise including at least one high-shear mixing stage in a multi-stage wet-milling sequence.

In certain embodiments, different pre-grinding moisture levels are used. The oat raw material may be pre-wetted to a specific moisture content, such as 10 to 30 weight percent, prior to the first grinding step in order to optimise energy efficiency and reduce dust formation. A method for preparing a slurry may therefore comprise pre-wetting oat raw material to a predetermined moisture level before grinding.

In addition to the high-pressure homogenisation steps described above, further embodiments relate to one-, two-, three- or four-stage homogenisation sequences of the slurry or the liquid oat base. A liquid oat base production method may therefore comprise subjecting a slurry to at least one and up to four successive homogenisation stages.

In one group of embodiments, a two-stage high-pressure homogenisation is used in which a first homogeniser stage operates at a first pressure P1 and a second homogeniser stage operates at a second pressure P2, where P2 is equal to or greater than P1. The first pressure may be selected from the range of 100 to 700 bar, 150 to 500 bar or 200 to 400 bar. The second pressure may be selected from the range of 300 to 1000 bar, 400 to 800 bar or 500 to 800 bar. The homogenisation may be carried out in a single homogeniser unit including two valves in series or in two separate homogeniser units. A method may therefore comprise homogenising a slurry in a first stage at a lower pressure and in a second stage at a higher pressure.

In some embodiments, the geometry of the homogenisation gap is specifically configured. The homogenising valve may comprise a converging channel, a restricted gap and a diverging channel. The restricted gap may have a height in the range of 10 to 300 micrometres, 20 to 150 micrometres or 40 to 100 micrometres. The length-to-height ratio of the restricted gap may be from 5 to 100, 10 to 50 or 15 to 40. The gap may be straight, wedge-shaped or curved, or may include micro-structures such as ribs, grooves, dimples or protrusions that promote controlled cavitation and turbulence. A homogenisation method may therefore comprise passing a slurry through a restricted gap having one of the above geometries.

In other embodiments, the homogeniser is equipped with interchangeable valve inserts having different geometries, including but not limited to radial-valve geometry, axial-valve geometry or a multi-orifice plate having an array of micro-orifices. The orifices may have diameters in the range of 50 to 500 micrometres, for example 80 to 300 micrometres. By selecting different inserts, the intensity and nature of shearing, impact and cavitation can be tailored. A method may therefore comprise selecting a valve insert from a set of interchangeable inserts to modify the flow characteristics through the homogenisation valve.

In some embodiments, the homogenisation pressure is modulated over time. The process may apply a stepped pressure profile in which the pressure is held at different levels for successive time periods, a ramped pressure profile in which the pressure increases or decreases gradually, or a pulsed pressure profile in which the pressure alternates between high and low phases. Such pressure modulation may enable a desired balance of particle-size reduction, viscosity development and energy consumption. A method may therefore comprise homogenising a slurry according to a predetermined pressure modulation pattern.

In a further group of embodiments, the homogeniser includes temperature-control means. The slurry or liquid oat base may be cooled or heated upstream or downstream of one or more homogenisation stages. Cooling may be applied to limit temperature rise at high pressure, whereas mild heating may be applied to adjust viscosity or enzyme activity. A homogenisation process may therefore comprise regulating the temperature of the slurry before or after homogenisation.

In some embodiments, energy-efficiency optimisation is achieved by selecting a combination of particle size after milling and total homogenisation pressure such that the energy consumption per unit of product is minimised while a target particle-size distribution such as a D90 between 100 and 150 micrometres is achieved. For example, a relatively coarse ground slurry having a D90 of approximately 150 micrometres may be homogenised at a lower maximum pressure, whereas a finer ground slurry having a D90 of approximately 100 micrometres may be homogenised at a higher maximum pressure, leading to comparable total energy input but different textural outcomes. A method may therefore comprise selecting homogenisation pressures based on the initial particle-size distribution of the slurry.

In some embodiments, the homogenisation includes a third and optionally a fourth homogenisation stage. These additional stages may be operated at pressures of 50 to 300 bar and may stabilise emulsified oil droplets, break remaining fibre agglomerates or stabilise the distribution of beta-glucan-containing fragments. These stages may be implemented downstream of ingredient addition. A method may therefore comprise subjecting a slurry to at least three homogenisation stages including a final stabilising stage.

In certain embodiments, at least one homogenisation stage is a micro-fluidic homogenisation stage in which the slurry is forced through a micro-channel network or a micro-orifice array at a pressure between 400 and 2000 bar, thereby generating intense microscale shear and cavitation. A method may therefore comprise homogenising a slurry in a micro-fluidic homogeniser.

In addition to the process sequence in which oat raw material and water are milled, enzymatically treated, homogenised and subjected to enzyme deactivation, alternative process architectures are envisaged. A method for producing a liquid oat base may therefore comprise performing the operations of grinding, enzymatic treatment, homogenisation and enzyme deactivation in alternative orders.

In one group of embodiments, the process sequence comprises a first grinding step or grinding sequence, a first enzyme treatment step, a second grinding step or grinding sequence, a two-stage high-pressure homogenisation and enzyme deactivation. In this architecture, a portion of the hydrolysis is allowed to proceed on relatively coarse particles, after which further grinding is carried out to expose additional surfaces to the enzymes. A process may therefore comprise enzymatically treating a partially ground slurry before completing the grinding sequence.

In another group of embodiments, the process comprises a first grinding sequence to form a slurry, a first high-pressure homogenisation step, an enzyme treatment step, a second high-pressure homogenisation step and enzyme deactivation. In this architecture, the first homogenisation step may be operated at a relatively low or moderate pressure to disperse particles and hydrate fibres, whereas the second homogenisation step may be operated at a higher pressure to achieve the desired final particle-size distribution and mouthfeel. A method may therefore comprise homogenising a slurry before and after enzyme treatment.

In yet another group of embodiments, the process comprises pre-heating water and oat raw material, performing an initial enzyme treatment at a relatively low temperature or low enzyme dosage, performing a three-stage wet-milling sequence, performing a second enzyme treatment at higher temperature or higher enzyme dosage, performing a two- or three-stage high-pressure homogenisation and performing enzyme deactivation. A method may therefore comprise carrying out multiple enzyme-treatment steps at different temperatures and dosages in combination with multi-stage milling and multi-stage homogenisation.

In some embodiments, intermediate heat treatments and coarse-solids separation steps may be inserted between the enzyme treatment and homogenisation steps to deactivate one subset of enzymes while leaving others active or to remove only very coarse particles before high-pressure homogenisation. A method may therefore comprise performing selective thermal treatments between process stages.

In certain embodiments, the entire method is implemented in a continuous processing line in which oat kernels or whole oat flour, water and enzymes are continuously fed, and in which the grinding stages, high-shear mixing stages, enzyme-treatment stages, homogenisation stages and heat-treatment stages are connected in series. A control device may receive signals from sensors monitoring flow rate, temperature, pressure, particle size and viscosity at different points in the line and may adjust operating parameters accordingly. A continuous method may therefore be controlled by automatic feedback from multiple sensors.

In other embodiments, the method is implemented in a semi-batch configuration in which grinding and enzymatic treatment are performed in batch vessels with recirculation, while homogenisation and final heat treatment are carried out continuously. A method may therefore comprise combining batch and continuous operations in a semi-batch process.

In a further aspect, embodiments relate to combined configurations of multi-stage wet-milling, multi-stage homogenisation and specific process sequences, together with process-control strategies. A comprehensive method for producing a liquid oat base may therefore include all these components.

In one group of embodiments, the combination includes a three-stage wet-milling sequence comprising a disc mill, a first colloid mill and a second colloid mill, configured to provide a slurry with a D90 of approximately 120 to 160 micrometres and high solids retention, a two-stage high-pressure homogenisation sequence at pressures of, for example, 200 to 400 bar and 500 to 800 bar, an enzyme treatment step performed before the first homogenisation, between the first and second homogenisation steps or after the second homogenisation step and a subsequent enzyme deactivation step. A method may therefore comprise combining multi-stage milling, multi-stage homogenisation and at least one enzyme treatment in any of the described orders.

In some embodiments, the order of the enzyme treatment and homogenisation steps is selected to optimise the balance between hydrolysis kinetics, particle-size reduction, viscosity development and energy consumption. For example, an enzyme treatment prior to homogenisation may favour rapid hydrolysis of starch in relatively large particles, whereas an enzyme treatment between homogenisation stages may promote more uniform conversion of partially fragmented particles. A method may therefore comprise adjusting the sequence of hydrolysis and homogenisation to achieve a desired processing outcome.

In some embodiments, the process is controlled by a control device comprising processor circuitry, memory and input and output interfaces. The control device may be configured to receive input signals from particle-size sensors, pressure sensors, temperature sensors, flow meters and viscosity sensors, calculate indicators of process performance including energy consumption per unit mass, particle-size reduction ratio, viscosity trajectory or solids-retention ratio and adjust operating parameters of the grinding, mixing, enzyme-treatment and homogenisation equipment based on these indicators. A method may therefore comprise controlling the production of a liquid oat base by automated adjustment of milling, enzymatic and homogenisation parameters.

If the measured D90 from the inline particle-size analyser after the last grinding stage exceeds a target value, the control device may reduce the disc-mill gap, increase rotor speed in a colloid mill, decrease feed rate or increase enzyme dosage in a subsequent hydrolysis step. Accordingly, a process may comprise modifying at least one grinding or enzymatic parameter when an inline measurement deviates from a target.

In some embodiments, a multi-objective optimisation is implemented in which the control device seeks to minimise energy consumption while respecting constraints including maximum allowed D90, minimum required solids retention and desired viscosity at a given solids content. The optimisation may be performed by iterative adjustment of pressure settings, mill gaps, flow rates and enzyme dosages. A method may therefore comprise performing multi-objective process optimisation during liquid oat base production.

In some embodiments, the process is configured such that no additional high-pressure homogenisation steps are applied beyond those explicitly defined for a particular embodiment and that no additional grinding steps beyond the defined multi-step grinding sequence are performed prior to homogenisation. This allows precise definition of process configurations that may be compared in terms of energy consumption and product properties. A method may therefore comprise limiting the process to specific grinding and homogenisation stages.

In a further aspect, embodiments relate to a system for producing a liquid oat base and a liquid oat product. The system comprises one or more grinding devices including at least one disc mill and at least one colloid mill connected in series, one or more enzyme-treatment vessels or reactors, at least one high-pressure homogeniser configured to perform one, two, three or more high-pressure homogenisation stages and optionally equipped with interchangeable valve inserts and controlled gap geometries, one or more heat-treatment units for enzyme deactivation and product sterilisation and a control device configured to operate the grinding devices, enzyme-treatment vessels, homogenisers and heat-treatment units according to any of the process sequences described herein. The system may further comprise one or more particle-size sensors, viscosity sensors, flow meters, temperature sensors or pressure sensors positioned at selected locations including upstream of the first grinding stage, between grinding stages, upstream and downstream of homogenisation stages and at the outlet of the line. A system claim may therefore define a production installation including grinding devices, homogenisers, enzyme-treatment reactors and a control device programmed to execute any of the methods described above.

The processing of the water and oat kernels in a three-step grinding sequence may consist of, in the following order and without any additional or intermediate grinding or milling steps: a first grinding step carried out in a first grinding device, a second grinding step carried out in a second grinding device, and a third grinding step carried out in a third grinding device, thereby obtaining a three-step ground slurry. The three-step ground slurry may be subjected to an enzyme-treatment step. The enzyme-treated slurry may be subjected to a two-step homogenisation sequence consisting of, in the following order and without any additional or intermediate homogenising or milling steps: a first homogenisation step, and a second homogenisation step.

## Claims

1. A method of producing a liquid oat base (7) for human consumption, said method comprising:
processing (12, 34) oat raw material, water (2), and one or more enzymes (5) into a slurry,
hydrolyzing (18) the slurry during a time period and by the one or more enzymes (5),
homogenizing (20) the slurry by high-pressure homogenization in a first homogenization step,
homogenizing (22) the homogenized slurry by high-pressure homogenization in a second homogenization step,
deactivating (24) the one or more enzymes in the two-step homogenized slurry, and
obtaining (33) the liquid oat base (7) from the two-step homogenized slurry.

2. The method of claim 1, wherein the slurry is produced to include at least 90%, 95% or 98% by weight of a solid content of the oat raw material.

3. The method of any preceding claim, wherein the two-step homogenized slurry has a particle size, given as D90, of less than 200 µm, or less than 150 µm.

4. The method of any preceding claim, wherein the two-step homogenized slurry has a particle size, given as D90, of at least 100 µm, or at least than 110 µm.

5. The method of any preceding claim, wherein the slurry is processed in the first homogenization step by high-pressure homogenization at a pressure of at least 100 bar (10 MPa), at least 200 bar (20 MPa), or at least 400 bar (40 MPa).

6. The method of any preceding claim, wherein the slurry is processed in the first homogenization step by high-pressure homogenization at a pressure of at most 700 bar (70 MPa), or at most 500 bar (50 MPa).

7. The method of any preceding claim, wherein the homogenized slurry is processed in the second homogenization step by high-pressure homogenization at a pressure of at least 300 bar (30 MPa), at least 500 bar (50 MPa), or at least 700 bar (70 MPa).

8. The method of any preceding claim, wherein the homogenized slurry is processed in the second homogenization step by high-pressure homogenization at a pressure of at most 1000 bar (100 MPa), or at most 800 bar (80 MPa).

9. The method of any preceding claim, wherein each of said
homogenizing (20) the slurry by high-pressure homogenization in a first homogenization step, and
homogenizing (22) the homogenized slurry by high-pressure homogenization in a second homogenization step,
comprises driving the respective slurry through a respective homogenization gap (G) with a size of 20-250 µm.

10. The method of any preceding claim, wherein said processing (12, 34) of oat raw material (3), water (2), and one or more enzymes (5) into a slurry comprises
grinding, in a first grinding step (14), at least the oat raw material (3) and the water (2) with a disc mill (62) into a one-step ground slurry.

11. The method of claim 10, comprising
grinding, in a second grinding step (15), the one-step ground slurry with a colloid mill (64) into a two-step ground slurry.

12. The method of claim 11, comprising
grinding, in a third grinding step (16), the two-step ground slurry with a further colloid mill (64) into a three-step ground slurry.

13. The method of any preceding claim, comprising no other high-pressure homogenization steps than the first homogenization step (20) and the second homogenization step, which are performed in series with no product-altering step in between.

14. A method of producing a liquid oat product (8) for human consumption, the method comprising
producing a liquid oat base (7) according to any preceding claim,
formulating (26) the oat base by mixing it with one or more ingredients (11),
homogenizing (30) the oat base and one or more ingredients by high-pressure homogenization in a third homogenization step, and
obtaining (33) the liquid oat product (8) from the homogenized oat base and one or more ingredients.

15. A system for producing a liquid oat base (7) for human consumption, said system comprising:
a first sub-system (91) configured to process (12, 34) oat raw material, water (2), and one or more enzymes (5) into a slurry,
a second sub-system (92) comprising one or more tanks (68) for receiving the slurry from the first sub-system (91), said second sub-system (92) being configured to hydrolyze the slurry during a time period and by use of the one or more enzymes (5),
a first homogenizer (70) configured to homogenize the slurry by high-pressure homogenization in a first homogenization step,
a second homogenizer (72) configured to homogenize the homogenized slurry by high-pressure homogenization in a second homogenization step,
a third sub-system (93) configured to deactivate the one or more enzymes in the two-step homogenized slurry, to thereby obtain the liquid oat base (7) from the two-step homogenized slurry.
